# EUROPEAN PATENT APPLICATION

(11) **EP 4 184 454 A1**
(43) Date of publication of application: **24.05.2023**
(21) Application number: 21209245.6
(22) Date of filing: 19.11.2021
(51) Int. Cl.: G06V 40/10, A61B 5/107, G01G 19/44, G06T 7/00

(54) **WEIGHT ESTIMATION OF A PATIENT**

(71) Applicant: Koninklijke Philips N.V., 5656 AG Eindhoven (NL)
(72) Inventor: SOMMER, Karsten, Eindhoven (NL); KRUEGER, Sascha, Eindhoven (NL); SENEGAS, Julien, Thomas, Eindhoven (NL); KOKEN, Peter, Eindhoven (NL)
(74) Representative: Philips Intellectual Property & Standards

(57) **Abstract**

A computer-implemented method is provided for estimating a weight of a patient when supported on a patient table. Optical image data and depth image data are obtained and patient body keypoints are extracted from the optical image data. A first frame is selected which comprises an image of the patient table, by selecting a frame in which no body keypoints are present in the optical image data. A second frame is selected of the patient on the table, by selecting a frame with patient body keypoints and with little or no movement. A patient volume is obtained based on a difference between the depth image data for the first and second frame (or depth data at those times) and the patient weight is estimated from the determined patient volume.

## Description

### FIELD OF THE INVENTION

This invention relates to the estimation of the weight of a subject, in particular a subject on a patient table, such as a patient undergoing a medical imaging examination.

### BACKGROUND OF THE INVENTION

Patient weight is an important parameter that is required for most medical imaging modalities.

In a clinical routine, patient metrics such as age, height, and weight are routinely recorded prior to medical imaging examinations such as magnetic resonance imaging, MRI, computer tomography, CT, imaging and positron emission tomography, PET, imaging. For each modality, patient weight is an important quantity: In MRI, safety-related specific absorption rate (SAR) calculations using patient weight as an input parameter are performed prior to each scan to prevent excessive radio frequency, RF, exposure. For CT examinations, weight-based tube currents can be used to minimize radiation dose. Moreover, in all modalities, determination of appropriate contrast agent volumes requires precise knowledge of the patient's weight.

One common approach is to question the patient about their weight. Another is simply to estimate the patient weight based on their visual appearance. However, these approaches may lead to substantial errors.

Using a scale to determine the weight provides accurate results, but introduces undesired complexity to the workflow. Moreover, depending on their medical condition, not every patient can be interviewed or positioned on a scale.

Contactless weight measurement systems have been proposed. For example EP 3699929 discloses a system for estimating a patient weight before medical examination using a depth camera. A patient model in the form of a mesh is fit to a depth image, and weight is estimated from extracted feature values derived from the patient model using regression analysis.

### SUMMARY OF THE INVENTION

The invention is defined by the claims.

According to examples in accordance with an aspect of the invention, there is provided a computer-implemented method for estimating a weight of a patient when supported on a patient table, comprising:
receiving image data comprising optical image data of an optical image of the patient and depth image data of a depth image of the patient, the image data comprising frames;
detecting patient body keypoints from the optical image data;
selecting a first frame which comprises an image of the patient table, by selecting a frame in which no patient body keypoints are present in the optical image data;
selecting a second frame which comprises an image of the patient on the table, by selecting a frame in which:
   the number of patient body keypoints present in the optical image data is greater than a first threshold; and
   a movement measure of the patient body keypoints in the optical image data between the frame and at least one previous frame is below a second threshold;
determining a patient volume based on a difference between the depth image data at the time of the first frame and the depth image data at the time of the second frame; and
estimating a patient weight from the determined patient volume.

This method provides a contactless patient weight estimation method, which can be seamlessly integrated into a clinical workflow. The method uses an optical image camera (e.g. an RGB image sensor) and a depth camera, which provides a video stream of the patient table area during examination preparation.

The invention enables a fully automatic and robust estimation of patient weight without requiring manual selection of the input camera data. Instead, the optimal frames for determining patient volume and hence weight are selected automatically, and this may take place during patient preparation.

The camera system is for example a ceiling-mounted RGB camera and depth camera to enable contactless weight determination. The weight estimation may be used as complementary information to the biometric data available via the electronic patient record, for example to confirm the information in the electronic patient record or to indicate a possible mismatch.

Detecting the patient body keypoints for example makes use of a neural network. Algorithms for analysis of image data to extract body keypoints are well known.

The method may comprise identifying a set of second frames comprising all frames in which the number of patient body keypoints present is greater than the first threshold and the movement measure of the patient body keypoints between the frame and the at least one previous frame is below the second threshold, and determining a patient volume for each of the frames of the second set.

In this way, multiple volume determinations may be made, so that a best one for use in estimating the patient weight may be selected. For example, estimating the patient weight may be based on the lowest determined patient volume.

Determining a patient volume for example comprises deriving a volume map from the depth data.

In a most basic approach, a typical patient density may be assumed (or one associated with the particular type (age, gender etc.) of patient in order to convert from volume to weight. However, a patient weight may instead be derived more accurately by using anatomical models of typical tissue distributions adapted to a patient volume map. The patient weight may for example be estimated by using a neural network with one or more volume maps as input.

The method may comprise receiving image data for a constant patient table position. In this way, the depth data for any second frame can be combined with the depth data for any first frame.

Alternatively, image data may be received for different patient table positions, and the method comprises determining a table position from the image data and calibrating the depth data using the determined table position. Thus, a correction may be made if different frames contain data for different table positions.

When image data is received for different patient table positions, the method may instead comprise selecting first and second frames corresponding to the same table position. For example, at a new table position, a new first frame may be required.

Determining a patient volume for example comprises measuring a volume of a portion of the patient's body using the difference between the depth image data, and extrapolating from the determined patient volume to the estimated patient weight. The volume estimation may for example be based only on central body parts such as the trunk, because limbs and the head are more likely to give inaccurate results.

The invention also provides a computer program comprising computer program code means which is adapted, when said program is run on a computer, to implement the method defined above.

The invention also provides a controller which is programed with the computer program define above.

The invention also provides a weight estimation system, comprising:
an optical camera;
a depth camera; and
the controller defined above for processing image data comprising optical image data from the optical camera and depth image data from the depth camera to estimate a patient weight.

The optical camera and the depth camera are for example ceiling-mounted units.

The invention also provides a medical imaging system, comprising:
a patent support;
a medical imaging unit; and
the weight estimation system define above.

The medical imaging unit for example comprises a CT scanner, an MRI scanner or a PET scanner.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method of estimating a patient weight;
Figure 2 shows an image based representation of the method;
Figure 3 shows various image patient measures to illustrate how the method operates; and
Figure 4 shows an example of an imaging system which may be used to provide the images for analysis using the method of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for estimating a weight of a patient when supported on a patient table. Optical image data and depth image data are obtained and patient body keypoints are extracted from the optical image data. A first frame is selected which comprises an image of the patient table, by selecting a frame in which no patient body keypoints are present in the optical image data. A second frame is selected of the patient on the table, by selecting a frame with patient body keypoints and with little or no movement. A patient volume is obtained based on a difference between the depth image data for the first and second frames (or more generally a difference between depth images taken at the approximate or exact times of those first and second frames) and the patient weight is estimated from the determined patient volume.

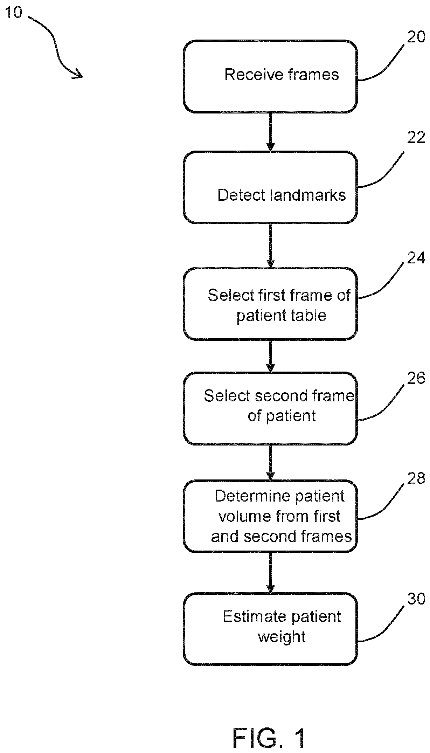

Figure 1 shows the method 10.

In step 20, image data is received comprising optical image data of an optical image of the patient and depth image data of a depth image of the patient. The image data is for example obtained from a ceiling-mounted camera system.

The image data comprises frames of optical data and frames of depth data. The frames may be the same, so that each frame comprises optical data and depth data. However, this is not essential, and the frame structures (e.g. frame rates and timings) may be different for the optical data and the depth data. Thus, independent cameras may be used.

The optical image data is for example data of 2D RGB camera images.

The depth image may be obtained by various possible camera designs. One example is a time-of-flight camera, in which the time of flight to travel to the object and back is measured. Alternatively, a stereo vision camera may be used, based on a pair of normal optical cameras mounted a certain distance apart. Structured light cameras are also known for 3D imaging. in which a scene is illuminated with specially designed light pattern.

Any of these options is possible for the depth camera.

The image data is received over a time period covering the initial patient preparation i.e. when they get onto a patient table of an imaging system.

In step 22, patient body keypoints are detected in the optical image data using known algorithms. The patient body keypoints are anatomical landmarks such as body joints or other anatomical features.

In step 24, a first frame is selected which comprises an image of the patient table, by selecting a frame in which no patient body keypoints are present in the optical image data. This frame may be considered to be an empty frame.

This first, empty, frame is selected to show a completely empty patient table, which is usually possible in the time between two imaging examinations. Fully automatic selection of this frame may be enabled using a dedicated neural network for body keypoint detection in step 22 that is applied to every frame of the camera optical image data stream (i.e. RGB data). The neutral network is "activated" when body keypoints are detected, and a level of neural network activation may thus be representative of a number of keypoints that has been detected.

By way of example, a set of keypoints may comprise 13 keypoints, including the 12 main joints (ankles, knees, hips, shoulders, elbows, wrists) and the head. By way of example, a "heatmap" of these keypoints may be generated, for example a 2D image with a Gaussian-like peak if a keypoint is detected.

To ensure that neither the operator nor the patient occlude the table, a thresholding criterion is applied to the network's total activation, for example ensuring the absence of any body keypoint from the image.

In one implementation, the remaining steps of the method rely on an assumption of an identical patient table height, so that any table motion logged by the imaging system may be used to trigger a renewed selection of this first, empty, frame.

Alternatively, detection of a patient table keypoint can be used to automatically infer the table position and to correct for possible table motion by applying the corresponding parameters (longitudinal and vertical translation) to the depth data, for example in combination with a camera calibration matrix.

In step 26, second frame is selected which comprises an image with the patient on the table. This second frame preferably shows the patient lying on the table, prior to the placement of coils or other accessories.

The second frame is selected for which the number of patient body keypoints present in the optical image data is greater than a first threshold. To implement this test of patient body keypoints, the total activation of the keypoint detection neural network in the image region of the table may be obtained for each frame of the optical image data stream. A minimum total activation is required, corresponding to this detection of a minimum number of keypoints. This ensures that the patient is really located on the table. Activations caused by the operator during examination preparation have been found not to produce a false-positive triggering of this criterion.

The selection of the second frame also involves making a movement measure of the patient body keypoints in the optical image data between the frame and at least one previous frame, for example between two successive frames of the optical image data. This movement measure must be below a second threshold.

In this way, frames with large patient movements are not selected as the second frame. The movement measure is derived as a metric of body keypoint motion, calculated by measuring the total frame-to-frame difference in body keypoint activations.

In step 28, a patient volume is obtained based on a difference between the depth image data for the first frame (or more generally depth image data corresponding as closely as possible to the timing of the first frame) and the depth image data for the second frame (or more generally depth image data corresponding as closely as possible to the timing of the second frame).

In a most basic approach only one first, empty, frame is selected and only one second, patient, frame is selected, and the volume is then obtained.

In a preferred approach, for all second frames that satisfy the two previous criteria, patient volume maps are calculated by subtracting the corresponding depth image from the depth image of the first, empty, frame.

A volume estimate is for example obtained by summation of all pixel values in the patient volume map. One most suitable second frame may then be selected i.e. one most suitable volume calculation. For example, the frame corresponding to the minimum volume estimate may be selected as the second, patient, frame. The rationale for this is based on the observation that depth camera based volume estimation is impaired in case of elevated body parts, which frequently occurs during the early stages of the patient preparation, or in case of occlusions due to accessories like MR coils or the operator.

When the frames of the optical image data and the depth image data are the same (i.e. there is a single camera system with a single frame timing), there is a direct correspondence between the frames identified by the optical analysis and the depth data to be used. However, if there is not a direct correspondence between the frames of optical image data and the frames of depth image data, time values may be identified for the frames selected as the first and second frames based on the analysis of keypoints, and then frames for the depth image data may be selected which most closely match the selected first and second frames in time. Thus, the patient volume is based on a difference between the depth image data at (or closest to) the timing of the first frame and the depth image data at (or closet to) the timing of the second frame.

In step 30, a patient weight is estimated from the determined patient volume. The patient weight may be estimated using a simple regression model. Calibration of this model may be performed by applying the algorithm to a range of subjects with known weights.

The patient weight may be estimated using anatomical models of typical tissue distributions that are adapted to the patient volume maps. These anatomical models can be based on the same body keypoints as used in the method.

The patient weight may be estimated using a neural network, using one or several patient volume map(s) as input. If reliable patient weight data is available at a clinical site (e.g. using a scale), such a system can even be trained and improved after deployment.

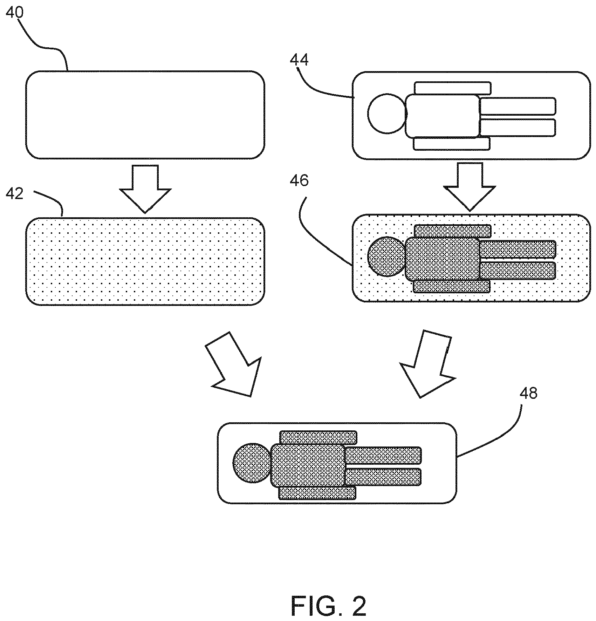

Figure 2 shows an image based representation of the method.

Image 40 represents the RGB camera image of the patient table for the selected first frame, and image 42 represents the depth image of the patient table for the selected first frame.

Image 44 represents the RGB camera image of the patient table and patient for the selected second frame, and image 46 represents the depth image of the patient table and patient for the selected second frame.

Image 48 represent the depth map based on a subtraction of image 46 from image 42 (or vice versa).

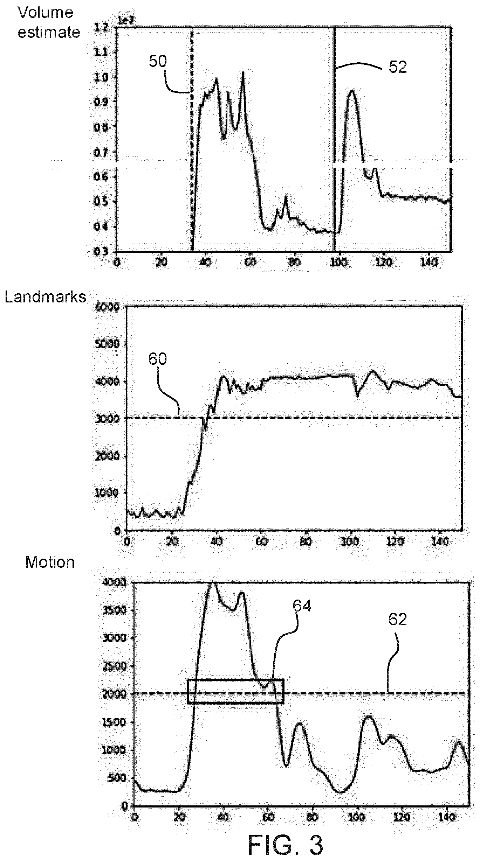

Figure 3 shows various patient measures to illustrate how the method operates..

Figure 3A shows a volume estimate (y-axis) versus frame number, wherein a volume estimate is made from each frame by comparing the depth image data with the depth image data for a first frame.

The first frame is obtained at the beginning, before there is any interaction with the patient table. For example, the first frame, selected as the empty frame, may be frame number 1.

The first non-zero volume estimation is at frame 35 as shown by line 50. However, at this time, the patient is just beginning to climb on the patient table. Thus, it is not a suitable frame for use as the second, patient, frame.

After frame 35, the patient is moving a lot, while getting into position on the patient table.

Figure 3B shows the activation level of the keypoint detection algorithm, representing how many keypoints are detected, as an arbitrary scale indicative of the number of reliable keypoints found.

A minimum neural network activation level is shown as the first threshold 60 (this threshold thus corresponds to a number of patient body keypoints present in the optical image data). If this criterion alone is used, frame 40 for example will erroneously selected as a suitable second, patient, frame. This is still in the phase where the patient is getting onto the table.

Figure 3C shows the motion measure based on the number of patient body keypoints in the optical image data which have moved (by an amount above a movement threshold) between the frame and the previous frame. The second threshold (of the number of moving keypoints) is shown as 62.

By using the additional patient motion criterion, the frames indicated by the box 64 are excluded, leading to an appropriate frame selection, such as frame 95 as shown by line 52 in Figure 3A.

This frame is the one for which the volume calculation in Figure 3A is a minimum, out of those frames that satisfy the two criteria for selection as the second frame.

For improved accuracy, especially in a case where the camera system optical axis is not orthogonal to the patient table plane, the depth data may first be transformed to 3D scanner coordinates using the camera calibration matrix, and the subtraction between the patient depth data and the patient table depth data may be performed in this Cartesian coordinate system.

Instead of relying purely on the RGB data for the automatic camera frame selection, other sensors such as infrared may be used.

The volume estimation may be performed only for the central body parts such as the trunk, because the limbs and the head are more prone to be elevated during examination preparation, leading to inaccurate results. The regression model may then be computed between this sub-volume and the whole patient weight.

Sanity checks may be performed to identify cases where the algorithm may have produced inaccurate results, e.g. if the estimated patient weight is very unusual for the detected patient dimensions (very high/low body-mass index). Especially, a quality check with biometric data available in the electronic patient record or in previous examinations can be performed and a significant mismatch can be communicated to the operator.

Additional standard image filters may be used to smooth or correct the depth image or the patient volume map.

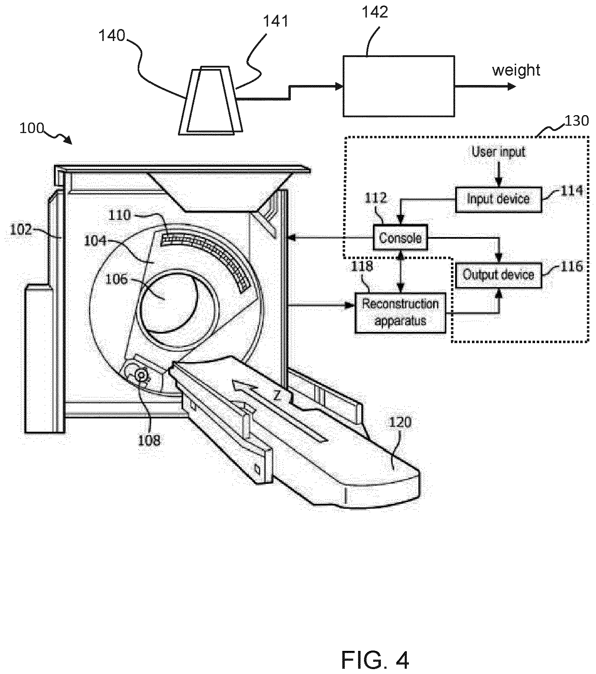

Figure 4 shows an example of an imaging system which may be used to provide the images for analysis using the method of the invention.

The imaging apparatus 100 in this illustration is an X-ray computed tomography (CT) scanner.

The imaging apparatus 100 includes a generally stationary gantry 102 and a rotating gantry 104. The rotating gantry 104 is rotatably supported by the stationary gantry 102 and rotates around an examination region about a longitudinal, axial, or z-axis.

A patient table 120, such as a couch, supports an object or subject such as a human patient in the examination region. The support 120 is configured to move the object or subject for loading, scanning, and/or unloading the object or subject. The support 120 is movable along an axial direction, that is, it is moveable along the direction of the z-axis or the longitudinal axis. Moving the support changes the axial position of the rotating gantry relative to the support (and thus relative to the subject who is supported by it).

A radiation source 108, such as an x-ray tube, is rotatably supported by the rotating gantry 104. The radiation source 108 rotates with the rotating gantry 104 and emits radiation that traverses the examination region 106.

A radiation sensitive detector array 110 subtends an angular arc opposite the radiation source 108 across the examination region 106. The detector array 110 includes one or more rows of detectors that extend along the z-axis direction, detects radiation traversing the examination region 106, and generates projection data indicative thereof.

The rotation of the gantry 104 changes an angular or rotational position of the scanner relative to the subject, and movement of the support along the z-axis changes the axial position of the scanner relative to the subject.

A typical scan will be configured in advance with a scan protocol. A scan protocol comprises a plurality of scanning parameters. The scanning parameters define, among other things, a spatial range of the scan relative to the axial and rotation axes of the scanner. For example, the scan parameters may include boundaries (that is, start and end points) of a scan range along one or more of the axes of the imaging apparatus, for example one or both of the rotational and axial axes. The scan range defines the field of view (FOV) over which imaging data is acquired during the scan. The scanning parameters may typically also include a number of other parameters including for example tube current, tube voltage, scan spatial resolution, scan temporal resolution, and/or fan angle. The resolution parameters may be defined by a speed of rotation of the gantry 104 and the speed of axial movement of the support 120 through the gantry.

A general-purpose computing system or computer serves as an operator console 112 and includes an input device(s) 114 such as a mouse, a keyboard, and/or the like and an output device(s) 116 such as a display monitor or the like. The console, input device(s) and output device(s) form a user interface 130. The console 112 allows an operator to control operation of the system 100.

A reconstruction apparatus 118 processes the projection data and reconstructs volumetric image data. The data can be displayed through one or more display monitors of the output device(s) 116.

The reconstruction apparatus 118 may employ a filtered-backprojection (FBP) reconstruction, a (image domain and/or projection domain) reduced noise reconstruction algorithm (e.g., an iterative reconstruction) and/or other algorithm. It is to be appreciated that the reconstruction apparatus 118 can be implemented through a microprocessor(s), which executes a computer readable instruction(s) encoded or embed on computer readable storage medium such as physical memory and other non-transitory medium. Additionally or alternatively, the microprocessor(s) can execute a computer readable instruction(s) carried by a carrier wave, a signal and other transitory (or non, non-transitory) medium.

The reconstruction apparatus 118 may incorporate a processor which is programmed with a computer program to implement the method described above, for analyzing the generated 3D CT scan image thereby to perform an analysis of the collateral vessels in the region of interest.

A ceiling mounted camera system is shown comprising an optical camera 140 and a depth camera 141. It provides image data, in the form of optical image data and depth image data, to a controller 142 which performs the method explained above, under the control of a suitable computer program,

The system thus makes use of a controller to perform the image data processing. The controller can be implemented in numerous ways, with software and/or hardware, to perform the various functions required. The controller typically employs one or more microprocessors that may be programmed using software (e.g., microcode) to perform the required functions. The controller may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

The invention can be used for contactless patient weight estimation during examinations preparations in medical imaging systems, including Magnetic Resonance (MR), Computer Tomography (CT), Positron Emission Tomography (PET)-CT, PET-MR, and MR-Linear Accelerator (Linac) systems. The estimated weight is for example used to derived a Specific Absorption Rate (SAR) value case of MR imaging or to derive recommended contrast agent volumes.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method for estimating a weight of a patient when supported on a patient table, comprising:
(20) receiving image data comprising optical image data of an optical image of the patient and depth image data of a depth image of the patient, the image data comprising frames;
(22) detecting patient body keypoints from the optical image data;
(24) selecting a first frame which comprises an image of the patient table, by selecting a frame in which no patient body keypoints are present in the optical image data;
(26) selecting a second frame which comprises an image of the patient on the table, by selecting a frame in which:
the number of patient body keypoints present in the optical image data is greater than a first threshold; and
a movement measure of the patient body keypoints in the optical image data between the frame and at least one previous frame is below a second threshold;
(28) determining a patient volume based on a difference between the depth image data at the time of the first frame and the depth image data at the time of the second frame; and
estimating a patient weight from the determined patient volume.

2. The method of claim 1, comprising:
identifying a set of second frames comprising all frames in which the number of patient body keypoints present is greater than the first threshold and the movement measure of the patient body keypoints between the frame and the at least one previous frame is below the second threshold; and
determining a patient volume for each of the frames of the second set.

3. The method of claim 2, wherein estimating the patient weight is based on the lowest determined patient volume.

4. The method of any one of claims 1 to 3, wherein determining a patient volume comprises deriving a volume map.

5. The method of claim 4, comprising estimating a patient weight by using anatomical models of typical tissue distributions adapted to a patient volume map.

6. The method of claim 4, comprising estimating a patient weight by using a neural network with one or more volume maps as input.

7. The method of any one of claims 1 to 6, comprising receiving image data for a constant patient table position.

8. The method of any one of claims 1 to 6, comprising receiving image data for different patient table positions, and the method comprises determining a table position from the image data and calibrating the depth data using the determined table position.

9. The method of any one of claims 1 to 6, comprising receiving image data for different patient table positions, and the method comprises selecting first and second frames corresponding to the same table position.

10. The method of any one of claims 1 to 9, wherein determining a patient volume comprises measuring a volume of a portion of the patient's body using the difference between the depth image data, and extrapolating from the determined patient volume to the estimated patient weight.

11. A computer program comprising computer program code means which is adapted, when said program is run on a computer, to implement the method of any one of claims 1 to 10.

12. A controller (142) which is programed with the computer program of claim 11.

13. A weight estimation system, comprising:
an optical camera (140);
a depth camera (141); and
the controller (142) of claim 12 for processing image data comprising the optical image data from the optical camera and depth image data from the depth camera to estimate a patient weight.

14. A medical imaging system, comprising:
a patent support;
a medical imaging unit; and
the weight estimation system of claim 13.

15. The medical imaging system of claim 14, wherein the medical imaging unit comprises a CT scanner, an MRI scanner or a PET scanner.
